# EUROPEAN PATENT APPLICATION

(11) **EP 2 977 367 A1**
(43) Date of publication of application: **27.01.2016**
(21) Application number: 14768024.3
(22) Date of filing: 14.03.2014
(51) Int. Cl.: C07C 67/08, C07C 69/54

(54) **PRODUCTION METHOD FOR (METH) ACRYLATE ARYL ESTER**

(30) Priority: 22.03.2013 JP 2013060459; 05.04.2013 JP 2013079070
(71) Applicant: Mitsubishi Rayon Co., Ltd., Tokyo 100-8253 (JP)
(72) Inventor: OBA, Fumi, Otake-shi Hiroshima 739-0693 (JP); MURATA, Naoshi, Otake-shi Hiroshima 739-0693 (JP); MORI, Hiroyuki, Otake-shi Hiroshima 739-0693 (JP)
(74) Representative: TBK
(86) International application number: PCT/JP2014/056893
(87) International publication number: WO 2014/148386

(57) **Abstract**

Provided is a low-tint (meth) acrylate aryl ester.

This production method for (meth) acrylate aryl ester causes a specific hydroxyl group-containing aromatic compound and a (meth) acrylate anhydride to react, in the presence of a hindered phenol and a specific phosphite, and produces a (meth) acrylate aryl ester.

## Description

### TECHNICAL FIELD

The present invention relates to a production method for a low-tint aryl (meth)acrylate.

### BACKGROUND ART

Using the high refractive index, low hygroscopicity, heat resistance, or the like of aryl group, aryl (meth)acrylate is generally useful for an application in various fields including plastics, a paint, adhesives, a treatment agent for paper processing, an oil agent for fibers, additives for a lubricant, a construction sealant, an ink, and the like. As a method for obtaining an aryl (meth)acrylate at high yield, a method of reacting (meth)acrylic acid anhydride with an aromatic compound containing a hydroxy group is known (Patent Documents 1 and 2).

### CITATION LIST

### PATENT DOCUMENT

Patent Document 1: JP 2010-126502 A
Patent Document 2: JP 5-140035 A

### DISCLOSURE OF THE INVENTION

### PROBLEM TO BE SOLVED BY THE INVENTION

However, for producing an aryl (meth)acrylate, there is a problem that coloration remains even in a product since the coloration by a reaction solution is high. According to the methods described in Patent Documents 1 and 2, the coloration cannot be reduced sufficiently so that a further improvement is in need.

An object of the present invention is to provide a low-tint aryl (meth)acrylate.

### MEANS FOR SOLVING PROBLEM

The present invention relates to the following (1) to (15).
(1) A production method for an aryl (meth)acrylate represented by the following formula [3] 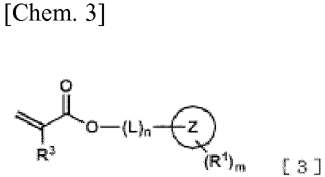 (in the formula, ring Z, L, R¹, n and m have the same meanings as defined in the following formula [1]. R³ represents a hydrogen atom or a methyl group), by reacting an aromatic compound containing a hydroxy group represented by the following formula [1] with (meth)acrylic acid anhydride 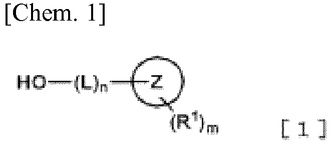 (in the formula, ring Z represents an aromatic hydrocarbon. L represents a divalent linking group. R¹ represents a linear or branched alkyl group, a cycloalkyl group, an aryl group, an aralkyl group, a carboxyl group, an alkoxycarbonyl group, an alkylcarbonyl group, an alkenylcarbonyl group, an arylcarbonyl group, a halogenated alkyl group, an alkoxy group, an aryloxy group, a trialkylsilyl group, a cyano group, a nitro group, a dialkylamino group, a sulfonic acid group, or a halogen atom. n represents an integer of 0 or 1 and m represents an integer of 0 or higher. When m is 2 or higher, R¹ may be the same or different from each other), in the presence of hindered phenol and phosphite represented by the following formula [2] 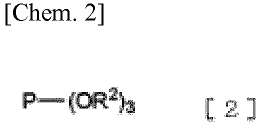 (in the formula, R² represents a linear or branched alkyl group, a cycloalkyl group, an aryl group, an aralkyl group, a carboxyl group, an alkoxycarbonyl group, an alkylcarbonyl group, an alkenylcarbonyl group, an arylcarbonyl group, or a halogenated alkyl group, which may have a substituent group or a hetero bond. R² may be the same or different from each other).
(2) The method described in (1), in which the reaction is performed in the presence of a catalyst and the ratio (%) of gas chromatography (GC) area value of the aromatic compound containing a hydroxy group to that of the aryl (meth)acrylate (aromatic compound containing a hydroxy group/ aryl (meth)acrylate x 100) is 13% or less after the reaction for 1 hour.
(3) The method described in (2), in which the catalyst is at least one of metal oxide and carbonate salt.
(4) The method described in (2), in which the catalyst is metal oxide, the reaction temperature is 80°C, and the metal oxide is allowed to be present at 0.006 equivalent or more relative to the aromatic compound containing a hydroxy group such that the ratio (%) of gas chromatography (GC) area value of the aromatic compound containing a hydroxy group to that of the aryl (meth)acrylate (aromatic compound containing a hydroxy group/-aryl (meth)acrylate x 100) is 13% or less after the reaction for 1 hour.
(5) A production method for an aryl (meth)acrylate represented by the following formula [3] 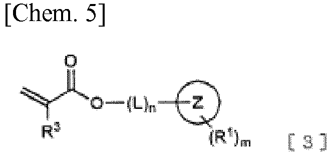 (in the formula, ring Z, L, R¹, n and m have the same meanings as defined in the following formula [1]. R³ represents a hydrogen atom or a methyl group)
   by reacting an aromatic compound containing a hydroxy group represented by the following formula [1] with (meth)acrylic acid anhydride in the presence of a catalyst, 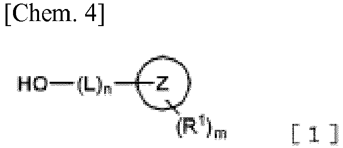 (in the formula, ring Z represents an aromatic hydrocarbon. L represents a divalent linking group. R¹ represents a linear or branched alkyl group, a cycloalkyl group, an aryl group, an aralkyl group, a carboxyl group, an alkoxycarbonyl group, an alkylcarbonyl group, an alkenylcarbonyl group, an arylcarbonyl group, a halogenated alkyl group, an alkoxy group, an aryloxy group, a trialkylsilyl group, a cyano group, a nitro group, a dialkylamino group, a sulfonic acid group, or a halogen atom. n represents an integer of 0 or 1 and m represents an integer of 0 or higher. When m is 2 or higher, R¹ may be the same or different from each other),
   in which the ratio (%) of gas chromatography (GC) area value of the aromatic compound containing a hydroxy group to that of the aryl (meth)acrylate (aromatic compound containing a hydroxy group/ aryl (meth)acrylate x 100) is 13% or less after the reaction for 1 hour.
(6) The method described in (5), in which the catalyst is at least one of metal oxide and carbonate salt.
(7) The method described in (5), in which the catalyst is metal oxide, the reaction temperature is 80°C, and the metal oxide is allowed to be present at 0.006 equivalent or more relative to the aromatic compound containing a hydroxy group such that the ratio (%) of gas chromatography (GC) area value of the aromatic compound containing a hydroxy group to that of the aryl (meth)acrylate (aromatic compound containing a hydroxy group/-aryl (meth)acrylate x 100) is 13% or less after the reaction for 1 hour.
(8) An aryl (meth)acrylate composition containing hindered phenol and phosphite represented by the following formula [2] 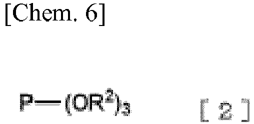 (in the formula, R² represents a linear or branched alkyl group, a cycloalkyl group, an aryl group, an aralkyl group, a carboxyl group, an alkoxycarbonyl group, an alkylcarbonyl group, an alkenylcarbonyl group, an arylcarbonyl group, or a halogenated alkyl group, which may have a substituent group or a hetero bond. R² may be the same or different from each other), each at 0.0002 parts by mass or more and 10 parts by mass or less relative to 100 parts by mass of aryl (meth)acrylate represented by the following formula [3] 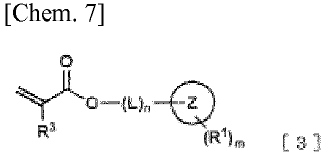 (in the formula, ring Z represents an aromatic hydrocarbon. L represents a divalent linking group. R¹ represents a linear or branched alkyl group, a cycloalkyl group, an aryl group, an aralkyl group, a carboxyl group, an alkoxycarbonyl group, an alkylcarbonyl group, an alkenylcarbonyl group, an arylcarbonyl group, a halogenated alkyl group, an alkoxy group, an aryloxy group, a trialkylsilyl group, a cyano group, a nitro group, a dialkylamino group, a sulfonic acid group, or a halogen atom. n represents an integer of 0 or 1 and m represents an integer of 0 or higher. When m is 2 or higher, R¹ may be the same or different from each other. R³ represents a hydrogen atom or a methyl group).
(9) An aryl (meth)acrylate composition containing aryl acetate represented by the following formula [4], 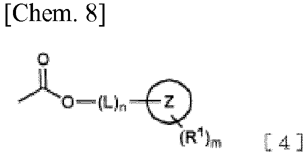 (in the formula, ring Z represents an aromatic hydrocarbon. L represents a divalent linking group. R¹ represents a linear or branched alkyl group, a cycloalkyl group, an aryl group, an aralkyl group, a carboxyl group, an alkoxycarbonyl group, an alkylcarbonyl group, an alkenylcarbonyl group, an arylcarbonyl group, a halogenated alkyl group, an alkoxy group, an aryloxy group, a trialkylsilyl group, a cyano group, a nitro group, a dialkylamino group, a sulfonic acid group, or a halogen atom. n represents an integer of 0 or 1 and m represents an integer of 0 or higher. When m is 2 or higher, R¹ may be the same or different from each other) and aryl (meth)acrylate represented by the following formula [3] 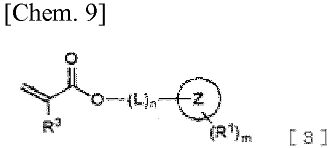 (in the formula, ring Z, L, R¹, n and m have the same meanings as defined in the formula [4]. R³ represents a hydrogen atom or a methyl group),
   in which the ratio (%) of gas chromatography (GC) area value of the aryl acetate to that of the aryl (meth)acrylate (aryl acetate/ aryl (meth)acrylate x 100) is 0.01 to 10%.
(10) A method for preserving aryl (meth)acrylate by containing, relative to 100 parts by mass of aryl (meth)acrylate represented by the following formula [3], 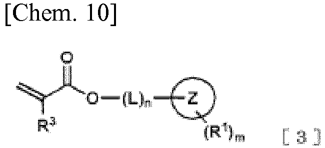 (in the formula, ring Z represents an aromatic hydrocarbon. L represents a divalent linking group. R¹ represents a linear or branched alkyl group, a cycloalkyl group, an aryl group, an aralkyl group, a carboxyl group, an alkoxycarbonyl group, an alkylcarbonyl group, an alkenylcarbonyl group, an arylcarbonyl group, a halogenated alkyl group, an alkoxy group, an aryloxy group, a trialkylsilyl group, a cyano group, a nitro group, a dialkylamino group, a sulfonic acid group, or a halogen atom. n represents an integer of 0 or 1 and m represents an integer of 0 or higher. When m is 2 or higher, R¹ may be the same or different from each other. R³ represents a hydrogen atom or a methyl group),
   hindered phenol and phosphite represented by the following formula [2]

   [Chem. 11] P-(OR²)₃ [2]

   (in the formula, R² represents a linear or branched alkyl group, a cycloalkyl group, an aryl group, an aralkyl group, a carboxyl group, an alkoxycarbonyl group, an alkylcarbonyl group, an alkenylcarbonyl group, an arylcarbonyl group, or a halogenated alkyl group, which may have a substituent group or a hetero bond. R² may be the same or different from each other),
   each at 0.0002 parts by mass or more and 10 parts by mass or less.
(11) An aryl (meth)acrylate composition containing an aryl (meth)acrylate represented by the following formula [3], in which a toluene solution of the composition has absorbance of 0.4 or less at a wavelength of 417 nm 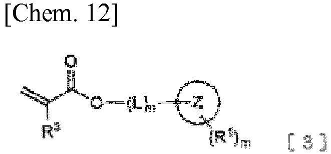 (in the formula, ring Z represents an aromatic hydrocarbon. L represents a divalent linking group. R¹ represents a linear or branched alkyl group, a cycloalkyl group, an aryl group, an aralkyl group, a carboxyl group, an alkoxycarbonyl group, an alkylcarbonyl group, an alkenylcarbonyl group, an arylcarbonyl group, a halogenated alkyl group, an alkoxy group, an aryloxy group, a trialkylsilyl group, a cyano group, a nitro group, a dialkylamino group, a sulfonic acid group, or a halogen atom. n represents an integer of 0 or 1 and m represents an integer of 0 or higher. When m is 2 or higher, R¹ may be the same or different from each other. R³ represents a hydrogen atom or a methyl group).
(12) The aryl (meth)acrylate composition described in (11), further containing an aryl acetate represented by the following formula [4], 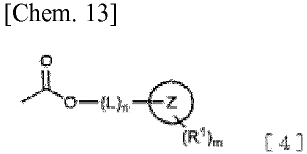 (in the formula, ring Z represents an aromatic hydrocarbon. L represents a divalent linking group. R¹ represents a linear or branched alkyl group, a cycloalkyl group, an aryl group, an aralkyl group, a carboxyl group, an alkoxycarbonyl group, an alkylcarbonyl group, an alkenylcarbonyl group, an arylcarbonyl group, a halogenated alkyl group, an alkoxy group, an aryloxy group, a trialkylsilyl group, a cyano group, a nitro group, a dialkylamino group, a sulfonic acid group, or a halogen atom. n represents an integer of 0 or 1 and m represents an integer of 0 or higher. When m is 2 or higher, R¹ may be the same or different from each other), and the ratio (%) of gas chromatography (GC) area value of the aryl acetate to that of the a ryl (meth)acrylate (aryl acetate/ aryl (meth)acrylate x 100) is 0.01 to 10%.
(13) The aryl (meth)acrylate composition described in (12), which further contains hindered phenol and phosphite represented by the following formula [2]

   [Chem. 14] P-(OR²)₃ [2]

   (in the formula, R² represents a linear or branched alkyl group, a cycloalkyl group, an aryl group, an aralkyl group, a carboxyl group, an alkoxycarbonyl group, an alkylcarbonyl group, an alkenylcarbonyl group, an arylcarbonyl group, or a halogenated alkyl group, which may have a substituent group or a hetero bond. R² may be the same or different from each other),
   each at 0.0002 parts by mass or more and 10 parts by mass or less relative to 100 parts by mass of aryl (meth)acrylate,
(14) An aryl (meth)acrylate composition containing an aryl (meth)acrylate represented by the following formula [3] and having the total chlorine content of 13000 ppm or less 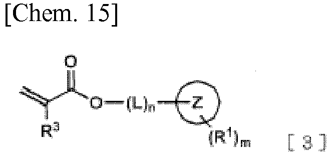 (in the formula, ring Z represents an aromatic hydrocarbon. L represents a divalent linking group. R¹ represents a linear or branched alkyl group, a cycloalkyl group, an aryl group, an aralkyl group, a carboxyl group, an alkoxycarbonyl group, an alkylcarbonyl group, an alkenylcarbonyl group, an arylcarbonyl group, a halogenated alkyl group, an alkoxy group, an aryloxy group, a trialkylsilyl group, a cyano group, a nitro group, a dialkylamino group, a sulfonic acid group, or a halogen atom. n represents an integer of 0 or 1 and m represents an integer of 0 or higher. When m is 2 or higher, R¹ may be the same or different from each other. R³ represents a hydrogen atom or a methyl group).
(15) The aryl (meth)acrylate composition described in (14), further containing an aryl acetate represented by the following formula [4], 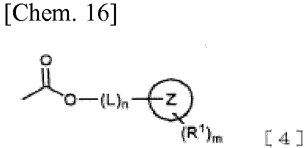 (in the formula, ring Z represents an aromatic hydrocarbon. L represents a divalent linking group. R¹ represents a linear or branched alkyl group, a cycloalkyl group, an aryl group, an aralkyl group, a carboxyl group, an alkoxycarbonyl group, an alkylcarbonyl group, an alkenylcarbonyl group, an arylcarbonyl group, a halogenated alkyl group, an alkoxy group, an aryloxy group, a trialkylsilyl group, a cyano group, a nitro group, a dialkylamino group, a sulfonic acid group, or a halogen atom. n represents an integer of 0 or 1 and m represents an integer of 0 or higher. When m is 2 or higher, R¹ may be the same or different from each other), and the ratio (%) of gas chromatography (GC) area value of the aryl acetate to that of the aryl (meth)acrylate (aryl acetate/(meth)acrylate aryl (meth)acrylate x 100) is 0.01 to 10%.

### EFFECT OF THE INVENTION

A low-tint aryl (meth)acrylate can be provided by the present invention.

### BRIEF DESCRIPTION OF DRAWINGS

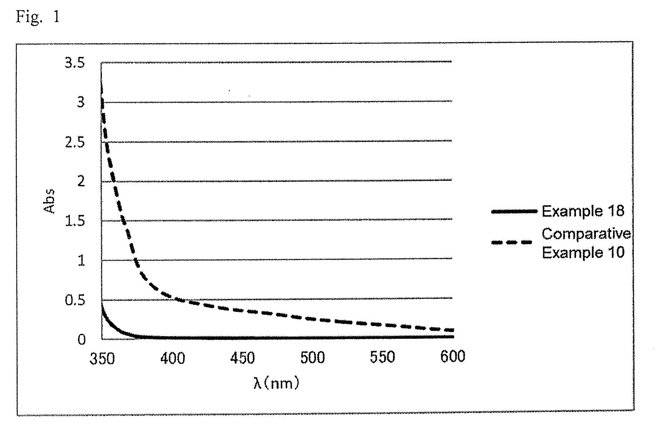

Fig. 1 is a graph showing the absorbance of a toluene solution of aryl (meth)acrylate of Example 18 and Comparative Example 10.

### MODE(S) FOR CARRYING OUT THE INVENTION

As a result of intensive studies, the inventors of the present invention found that a low-tint aryl (meth)acrylate can be produced by reacting an aromatic compound containing a hydroxy group that is represented by the formula [1] with (meth)acrylic acid anhydride in the presence of a specific anti-oxidant and/or a catalyst. It was also found that an aryl (meth)acrylate composition containing a specific anti-oxidant or aryl acetate has excellent storage stability and low tint.

In the present specification, acrylate and methacrylate are collectively described as (meth)acrylate. Acrylic acid anhydride and methacrylic acid anhydride are collectively described as (meth)acrylic acid anhydride. Further, (meth)acrylic acid anhydride is also described as anhydrous (meth)acrylic acid.

### [Production method for aryl (meth)acrylate]

According to the production method for an aryl (meth)acrylate of the present invention, an aromatic compound containing a hydroxy group represented by the above formula [1] and (meth)acrylic acid anhydride are reacted with each other.

The aromatic compound containing a hydroxy group which is used in the present invention is represented by the above formula [1]. As described herein, ring Z represents an aromatic hydrocarbon. Examples of the aromatic hydrocarbon include benzene, naphthalene, azulene, anthracene, phenanthrene, pyrene, and chrysene. Among them, from the viewpoint of easy industrial availability due to low price, benzene and naphthalene are preferred as an aromatic hydrocarbon. Further, from the viewpoint of having high refractive index, low hygroscopicity, and excellent heat resistance or the like, naphthalene is preferred more as an aromatic hydrocarbon.

Examples of the divalent linking group represented by L include a divalent linking group which may have a substituent group or a hetero bond. Examples of the divalent linking group include a linear, branched, or cyclic alkylene group, an alkenylene group, and an alkynylene group. Specific examples thereof include a methylene group, an ethylene group, a propylene group, a butylene group, an isopropylene group, a cyclohexylene group, and a combination thereof.

Examples of the divalent linking group which has a hetero bond include an ether, an ester bond, a thio ether bond, a thio ester bond, a sulfonyl bond, an amide bond, an imide bond, a sulfonamide bond, a urethane bond, a urea bond, a thiourea bond, and a divalent alkylene group having those bonds. Meanwhile, the hetero bond may be present either inside or at the end of a divalent linking group. Further, only one or plural hetero bonds may be present in a divalent linking group. Further, the divalent linking group may consist of a hetero bond. n is an integer of 0 or 1. Meanwhile, L may contain a structure in which a common divalent linking group is repeated. In such case, from the viewpoint of maintaining properties of an aryl (meth)acrylate like high refractive index, low hygroscopicity, and heat resistance, number of the repetition is preferably 2 to 20, more preferably 2 to 10, and even more preferably 2 to 5.

Examples of the substituent group R¹ include a linear or branched alkyl group such as a methyl group, an ethyl group, a propyl group, an isopropyl group, a butyl group, a s-butyl group, or a t-butyl group; a cycloalkyl group such as a cyclopentyl group or a cyclohexyl group; a phenyl group, an alkylphenyl group such as a 4-methylphenyl group, dimethylphenyl group, and an aryl group such as a naphthyl group; an aralkyl group such as a benzyl group or a phenethyl group; a carboxy group; an alkoxycarbonyl group such as a methoxycarbonyl group; an alkylcarbonyl group such as an acetyl group; an alkenylcarbonyl group; an arylcarbonyl group such as a phenylcarbonyl group; a halogenated alkyl group such as a trifluoromethyl group; an alkoxy group such as a methoxy group, an ethoxy group, a propoxy group, a n-butoxy group, an isobutoxy group, or a t-butoxy group; an aryloxy group such as a phenyloxy group or a 2-methylphenyloxy group; a trialkylsilyl group such as a trimethylsilyl group; a cyano group; a nitro group; a dialkylamino group such as a diethylamino group; a sulfonic acid group; and a halogen atom such as a fluorine atom, a chlorine atom, or a bromine atom. In the formula [1], when m is 2 or higher, R¹ may be the same or different from each other. The substitution position of R¹ in the ring Z is not particularly limited. When m is an integer of 0 or higher, it may be appropriately selected depending on the ring number of the ring Z, and it is not particularly limited. For example, when the ring Z is a naphthalene ring, from the viewpoint of easy obtainability of a raw material, m is preferably 0 to 4, more preferably 0 to 2, and even more preferably 0 or 1.

Examples of the aromatic compound containing a hydroxy group represented by the above formula [1] include 2-naphthol, 1-naphthol, 2-methyl-1-naphthol, 3-fluoro-2-naphthol, 4-phenyl-1-naphthol, methyl 4-hydroxynaphthalene-1-carboxylate, 4-hydroxy-1-naphthylmethyl ketone, 4-methoxy-1-naphthol, 4-phenoxy-1-naphthol, 1-nitro-2-naphthol, 4-dimethylamino-1-naphthol, 2-naphthalene methanol, phenol, o-cresol, 2,4-dimethylphenol, 2-fluorophenol, 4-phenylphenol, 4-hydroxyphenyl methyl ketone, 4-methoxyphenol, 4-phenoxyphenol, 4-cyanophenol, 9-hydroxymethylanthracene, and 1-pyrene methanol. It may be used either singly or in combination of two or more types.

As for the (meth)acrylic acid anhydride, a commercially available product or a separately synthesized product can be used. The use amount of the (meth)acrylic acid anhydride which is used in the present invention is not particularly limited. However, from the viewpoint of obtaining an aryl (meth)acrylate at good yield, the use amount of the (meth)acrylic acid anhydride is preferably 0.5 mol or more per 1 mol of the hydroxy group of an aromatic compound containing a hydroxy group. More preferably, it is 0.7 mol or more. Even more preferably, it is 0.9 mol or more. Further, from the viewpoint of preventing polymerization originating from (meth)acrylic acid anhydride, the use amount of the (meth)acrylic acid anhydride is preferably 2 mol or less per 1 mol of the hydroxy group of an aromatic compound containing a hydroxy group. More preferably, it is 1.8 mol or less. Even more preferably, it is 1.5 mol or less.

According to the present invention, a low-tint aryl (meth)acrylate can be produced by performing the reaction in the presence of hindered phenol and phosphite represented by the formula [2]. The hindered phenol has an effect of supplementing peroxy radicals that are produced by oxidation of an aromatic compound containing a hydroxy group. Further, the phosphite represented by the formula [2] has an effect of decomposing hydroperoxide that is produced by an action of hindered phenol. In particular, since an effect of suppressing generation of a quinone structure substance, which is a tint-causing substance, is obtained by using them in combination in the present invention, a low-tint aryl (meth)acrylate can be obtained.

As described herein, the hindered phenol has a bulky substituent group at two ortho positions of a phenolic hydroxy group in addition to a hydrogen atom and a methyl group. Example of the bulky substituent group include an alkyl group other than a methyl group, an alkenyl group, an alkynyl group, an aryl group, a heterocyclic group, an alkoxy group, an aryloxy group, a substituted amino group, a thioalkyl group, and a thiophenyl group. Examples of the hindered phenol include 2,6-di-tert-butylphenol, 2,6-di-tert-butyl-4-methylphenol, 2,4,6-tri-tert-butylphenol, 4,4'-methylenebis(2,6-di-t-butylphenol), 2,6-di-tert-butyl-4-(octadecaneoxycarbonylethyl)phenol, and 1,3,5-dimethyl-2,4,6-tris(3,5-tert-butyl-4-hydroxybenzyl)benzene. It may be used either singly or in combination of two or more types.

Examples of the substituent group R² of phosphite represented by the above formula [2] include a linear or branched alkyl group such as a methyl group, an ethyl group, a propyl group, an isopropyl group, a butyl group, a s-butyl group, a t-butyl group, a 2-ethylhexyl group, a decyl group, a lauryl group, or an oleyl group; a cycloalkyl group such as a cyclopentyl group or a cyclohexyl group; a phenyl group, an alkylphenyl group such as a 4-methylphenyl group, and an aryl group such as a dimethylphenyl group and a naphthyl group; an aralkyl group such as a benzyl group and a phenethyl group; a carboxy group; an alkoxycarbonyl group such as a methoxycarbonyl group; an alkylcarbonyl group such as an acetyl group; an alkenylcarbonyl group; an arylcarbonyl group such as a phenylcarbonyl group; and a halogenated alkyl group such as a trifluoromethyl group. R² may be the same or different from each other. Further, R² may have a substituent group or a hetero bond. Examples of the hetero bond include an ether bond, an ester bond, a phosphate ester bond, a phosphite ester bond, a phosphonate ester bond, a thio ether bond, a thio ester bond, a sulfonyl bond, an amide bond, an imide bone, a sulfonamide bond, a urethane bond, a urea bond, and a thiourea bond. Meanwhile, the hetero bond may be present either inside or at the end of the substituent group R². Further, only one or plural hetero bonds may be present in the substituent group R². Further, diphosphite in which two phosphites that are represented by the formula [2] are bonded to each other via R² is also included in the phosphite that is represented by the formula [2] of the present invention. Examples of the phosphite represented by the above formula [2] include triethyl phosphite, tridecyl phosphite, trioleyl phosphite, triphenyl phosphite, tricresyl phosphite, tris(2,4-di-tert-butylphenyl)phosphite, diphenyl monodecyl phosphite, tetraphenyl dipropylene glycol diphosphite, bis(decyl)pentaerythritol diphosphite, distearyl pentaerythritol diphosphite, and bis(2,6-di-tert-butyl-4-methylphenyl)pentaerythritol diphosphite. It may be used either singly or in combination of two or more types.

In the present invention, the use amount of the hindered phenol is preferably 0.005 part by mass or more per 100 parts by mass of the aromatic compound containing a hydroxy group from the viewpoint of exhibiting a sufficient effect. It is more preferably 0.01 part by mass or more. Further, from the viewpoint of not having any influence on the physical properties of a polymer, the use amount of the hindered phenol is preferably 0.5 part by mass or less per 100 parts by mass of the aromatic compound containing a hydroxy group. More preferably, it is 0.3 part by mass or less.

The use amount of the phosphite that is represented by the above formula [2] is preferably 0.005 part by mass or more per 100 parts by mass of the aromatic compound containing a hydroxy group from the viewpoint of exhibiting a sufficient effect. It is more preferably 0.01 part by mass or more. Further, from the viewpoint of not having any influence on the physical properties of a polymer, the use amount of the phosphite that is represented by the above formula [2] is preferably 0.5 part by mass or less per 100 parts by mass of the aromatic compound containing a hydroxy group. More preferably, it is 0.3 part by mass or less.

The mass ratio between the hindered phenol and the phosphite represented by the formula [2] is, from the viewpoint of obtaining a high effect of suppressing coloration, preferably 10 : 90 to 90 : 10 in terms of hindered phenol: phosphite represented by the formula [2]. It is more preferably 25 : 75 to 75 : 25, and even more preferably 40 : 60 to 60 : 40.

According to the present invention, a low-tint aryl (meth)acrylate can be produced as an esterification is performed in the presence of a catalyst and the ratio (%) of gas chromatography (GC) area value of the aromatic compound containing a hydroxy group to that of the aryl (meth)acrylate (aromatic compound containing a hydroxy group/ aryl (meth)acrylate x 100) is adjusted to 13% or less after the reaction for 1 hour for suppressing oxidation of the aromatic compound containing a hydroxy group. Thus, it is preferable to use a catalyst allowing the aforementioned ratio of 13 % or less for the reaction. The ratio is more preferably 10% or less. Even more preferably, it is 8% or less. Particularly preferably, it is 5% or less. Meanwhile, the lower ratio is preferred more, and it can be 0%. Further, with regard to the reaction start time, the time point at which the internal temperature of a reaction vessel reaches the predetermined reaction temperature is employed as start time.

Examples of the catalyst capable of satisfying the above ratio range include a metal compound, an acid catalyst, a base catalyst, and a heterogeneous catalyst. Examples of the metal compound include metal oxide, metal hydroxide, metal chloride, metal inorganic acid salt such as metal carbonate salt, metal sulfate salt, metal nitrate salt, metal phosphate salt, or metal borate salt, metal organic acid salt such as metal carboxylate salt or metal sulfonic acid salt, metal acetylaceontate, and metal complex salt such as a metal cyclopentadienyl complex. Examples of the acid catalyst include inorganic acid such as sulfuric acid, nitric acid, phosphoric acid, boric acid, hydrochloric acid, and heteropoly acid; and organic acid such as methane sulfonic acid, p-toluenesulfonic acid, or camphor sulfonic acid. Examples of the base catalyst include an organic amine such as pyridine, 4-(dimethylamino)pyridine, or triethylamine. Examples of the heterogeneous catalyst include an ion exchange resin such as basic ion exchange resin or an acidic ion exchange resin and a catalyst having an active component supported on a carrier such as silica, alumina, or titania. The catalyst may be used either singly or in combination of two or more types. From the viewpoint of easily satisfying the aforementioned ratio range and having high operability, at least one of metal oxide and carbonate salt is preferred as a catalyst. Among them, from the viewpoint of easy obtainability, metal oxide or carbonate salt containing an alkali metal or an alkali earth metal is preferred as a catalyst. Examples of the meal oxide containing an alkali metal or an alkali earth metal include lithium oxide, sodium oxide, potassium oxide, magnesium oxide, calcium oxide, and barium oxide. Examples of the carbonate salt containing an alkali metal or an alkali earth metal include lithium carbonate, sodium carbonate, potassium carbonate, magnesium carbonate, calcium carbonate, and barium carbonate. Further, from the viewpoint of producing a low-tint aryl (meth)acrylate in a smaller amount and suppressing cost increase, load increase during a treatment step after the reaction, progress of a side reaction, or the like, magnesium oxide is preferred as a catalyst.

When metal oxide is used as a catalyst and the reaction temperature is 80°C, the metal oxide is preferably allowed to be present at 0.006 equivalent or more relative to the aromatic compound containing a hydroxy group so that the aforementioned ratio is 13% or less. More preferably, it is allowed to be present at 0.0065 equivalent or more. Meanwhile, from the viewpoint suppressing a side reaction or a load during a treatment step after the reaction, metal oxide is allowed to be present at 1 equivalent or less relative to the aromatic compound containing a hydroxy group. More preferably, it is allowed to be present at 0.5 equivalent or less.

With regard to the production method for an aryl (meth)acrylate of the present invention, the hindered phenol allowed to be present in the system also functions as a polymerization inhibitor. For further inhibition of a polymerization reaction, a polymerization inhibitor other than the hindered phenol may be additionally present. Examples of the polymerization inhibitor include, although not particularly limited, hydroquinone monomethyl ether, hydroquinone, 4-hydroxy-2,2,6,6-tetramethylpiperidine-N-oxyl, phenothiazine, and copper salt. It may be used either singly or in combination of two or more types. The use amount of the polymerization inhibitor is preferably 0.005 part by mass or more per 100 parts by mass of the aromatic compound containing a hydroxy group. More preferably, it is 0.01 part by mass or more. Further, the use amount of the polymerization inhibitor is preferably 0.5 part by mass or less per 100 parts by mass of the aromatic compound containing a hydroxy group. More preferably, it is 0.3 part by mass or less.

The reaction temperature for the esterification is preferably 25°C or higher from the viewpoint of shortening the reaction time. More preferably, it is 40°C or higher. Even more preferably, it is 60°C or higher. Particularly preferably, it is 70°C or higher. Further, from the viewpoint of suppressing a side reaction like a polymerization reaction, the reaction temperature is preferably 150°C or lower. More preferably, it is 110°C or lower. Even more preferably, it is 100°C or lower. Particularly preferably, it is 90°C or lower. The reaction pressure at the time of performing the esterification is not particularly limited. It can be performed under any pressure including reduced pressure, normal pressure, and increased pressure.

The reaction time for the esterification can be suitably selected depending on reaction conditions or the like. It may be 0.5 to 20 hours, for example.

For the production method of the present invention, it is not particularly necessary to use a reaction solvent. However, for a case in which the solubility of the aromatic compound containing a hydroxy group is low in the (meth)acrylic acid anhydride or (meth)acrylic acid that is produced over time, the viscosity of the reaction system is high, or it is desired to prevent the polymerization reaction, a reaction solvent can be suitably used. As for the reaction solvent, a solvent not containing active hydrogen can be used. Examples of such solvent include an aromatic solvent such as toluene or xylene; a hydrocarbon solvent such as hexane, heptane, octane, or cyclohexane; a polar solvent such as dimethyl formamide, dimethyl sulfoxide or acetonitrile, an ether solvent such as diethyl ether, diisopropyl ether, t-butyl methyl ether, tetrahydrofuran, or dioxane; and a halogen solvent such as chloroform or dichloromethane. The solvent may be used either singly or as a mixture. Further, the use amount can be suitably determined.

From the viewpoint of preventing polymerization between aryl (meth)acrylate and (meth)acrylic acid anhydride, the reaction is preferably performed in an air atmosphere. In particular, when metal oxide is used as a catalyst, a low-tint aryl (meth)acrylate can be sufficiently obtained even when the reaction is performed in an air atmosphere.

With regard to a method for purifying a reaction solution after completion of the esterification according to the present invention, washing like alkali washing and a purification method such as distillation, crystallization, or precipitation can be suitably combined in consideration of the physical properties of a product, type and amount of the raw materials and catalyst, presence or absence of a solvent, or the like.

### [Aryl (meth)acrylate composition]

The aryl (meth)acrylate composition according to the present invention contains hindered phenol and the phosphite represented by the formula [2], each at 0.0002 parts by mass or more and 10 parts by mass or less relative to 100 parts by mass of aryl (meth)acrylate represented by the formula [3]. It is preferable that the aryl (meth)acrylate composition contain hindered phenol and the phosphite represented by the formula [2], each at 0.0005 parts by mass or more and 5 parts by mass or less. More preferably, it contains each at 0.001 part by mass or more and 2 parts by mass or less. Even more preferably, it contains each at 0.005 part by mass or more and 0.5 part by mass or less. Particularly preferably, it contains each at 0.01 part by mass or more and 0.1 part by mass or less. As the aryl (meth)acrylate composition contains hindered phenol and the phosphite represented by the formula [2], each at 0.0002 parts by mass or more, coloration does not increase during storage, and thus good storage stability is obtained. Further, as the aryl (meth)acrylate composition contains hindered phenol and the phosphite represented by the formula [2], each at 10 parts by mass or less, an influence on physical properties of a polymer can be suppressed. The composition can be produced by controlling the amount of the hindered phenol and the phosphite represented by the formula [2] in the production method for an aryl (meth)acrylate. Further, the composition may be produced by adding the hindered phenol and the phosphite represented by the formula [2] in an amount within the aforementioned range relative to the aryl (meth)acrylate represented by the formula [3]. Further, the composition may also contain other components including a solvent and a monomer like methyl methacrylate (MMA). However, the amount of the aryl (meth)acrylate represented by the formula [3] contained in the composition is preferably 5% by mass or more. It is more preferably 10% by mass or more. It is even more preferably 15% by mass or more.

The aryl (meth)acrylate composition according to the present invention contains aryl acetate represented by the formula [4] and (meth)acrylate ester represented by the formula [3] and the ratio (%) of gas chromatography (GC) area value of the aryl acetate to that of the aryl (meth)acrylate (aryl acetate/ aryl (meth)acrylate x 100) is 0.01 to 10%. The ratio is more preferably 0.05 to 8%. Even more preferably, it is 0.1 to 5%. As the ratio is 0.01% or higher, the composition can have low tint. Further, as the ratio is 10% or less, an influence on physical properties of a polymer can be suppressed. The composition can be produced by adding aryl acetate represented by the formula [4] in an amount within the aforementioned range to (meth)acrylate represented by the formula [3]. Further, the composition can be also produced by performing the aforementioned production method for an aryl (meth)acrylate in the presence of an acetic acid component like acetic anhydride.

The aryl (meth)acrylate composition according to the present invention contains aryl (meth)acrylate represented by the formula [3], and the toluene solution of the composition has absorbance of 0.4 or less at wavelength of 417 nm. The absorbance is preferably 0.3 or less. More preferably, it is 0.2 or less. Even more preferably, it is 0.1 or less. Particularly preferably, it is 0.08 or less. Meanwhile, the lower absorbance is preferred more and it can be 0. As the absorbance is 0.4 or less, polymer coloration can be suppressed and the composition can be applied for use in which colorless transparency is required. The composition can be preferably produced by the aforementioned production method for aryl (meth)acrylate. Further, the absorbance indicates a value which is obtained by dissolving 1.00 g of the aryl (meth)acrylate composition in toluene to give 10 mL solution and absorbance at wavelength of 417 nm is determined by using a quartz cell (1 cm width and height) and a UV visible spectrophotometer (trade name: UV-1700, manufactured by Shimadzu Corporation).

The aryl (meth)acrylate according to the present invention contains (meth)acrylate represented by the formula [3] and it has total chlorine content of 13000 ppm or less. The total chlorine content is preferably 10000 ppm or less. It is more preferably 7000 ppm or less. Meanwhile, the less total chlorine content is preferred more and it can be 0 ppm. As the total chlorine content is 13000 ppm or less, the chlorine content contained in the polymer is reduced so that release of a chlorine component by a combustion treatment or the like is suppressed. As a result, a load on environment can be reduced. The composition can be preferably produced by the aforementioned production method for an aryl (meth)acrylate. Further, the total chlorine content is a value that is measured as follows; a distillation eluent is combusted by heating it to 100 to 900°C using a sample combustion device QF-02 (trade name, manufactured by Mitsubishi Chemical Corporation), and after allowing the gas to get absorbed in an absorbing pipe, analysis is performed by IC (ion chromatography).

By taking advantage of the high refractive index, low hygroscopicity, heat resistance, or the like that are possessed by an aryl group, the aryl (meth)acrylate composition according to the present invention can be used for various uses in many fields including plastics, a paint, adhesives, a treatment agent for paper processing, an oil agent for fibers, additives for a lubricant, a construction sealant, and an ink. Among them, by having low tint and low chlorine content, it can be preferably used for optical use or use for electronic materials requiring colorless transparency or less load on environment.

### [Method for preserving aryl (meth)acrylate]

According to the method for preserving an aryl (meth)acrylate of the present invention, hindered phenol and the phosphite represented by the formula [2] are contained each at 0.0002 parts by mass or more and 10 parts by mass or less relative to 100 parts by mass of aryl (meth)acrylate represented by the formula [3]. It is more preferable that hindered phenol and the phosphite represented by the formula [2] be contained each at 0.0005 parts by mass or more and 5 parts by mass or less. It is more preferable that hindered phenol and the phosphite represented by the formula [2] be contained each at 0.001 part by mass or more and 2 parts by mass or less. It is even more preferable that they be contained each at 0.005 part by mass or more and 0.5 part by mass or less. It is particularly preferable that they be contained each at 0.01 part by mass or more and 0.1 part by mass or less. As the hindered phenol and the phosphite represented by the formula [2] are contained each at 0.0002 parts by mass or more, coloration does not increase during storage, and thus good storage stability is obtained. Further, as the hindered phenol and the phosphite represented by the formula [2] are contained each at 10 parts by mass or less, an influence on physical properties of a polymer can be suppressed. The aryl (meth)acrylate may also contain other components including a solvent and a monomer like methyl methacrylate (MMA). However, the content of the aryl (meth)acrylate represented by the formula [3] is preferably 5% by mass or more. It is more preferably 10% by mass or more. It is even more preferably 15% by mass or more.

The container for storage can be any form including a glass container, a resin container, a metallic storage tank, a drum can, and a lorry.

Adjustment of the amount of hindered phenol and phosphite for satisfying the aforementioned mass ratio can be performed by adding a deficit amount, or by controlling reaction conditions or the like to have desired composition during the preparation of the aryl (meth)acrylate represented by the formula [3].

When a solvent or the like is present, the temperature for storage can be suitably determined depending on a solidification point. However, from the viewpoint of suppressing an energy load on cooling facilities, the temperature for storage is preferably -40°C or higher. It is more preferably -20°C or higher. It is even more preferably -10°C or higher. It is particularly preferably 0°C or higher. Further, from the viewpoint of suppressing a side reaction like a polymerization reaction, the temperature for storage is preferably 120°C or lower. It is more preferably 110°C or lower. It is even more preferably 100°C or lower.

### EXAMPLES

Hereinbelow, the present invention is described in detail in view of the examples. However, the present invention is not limited to those examples.

The reaction was followed by gas chromatography (GC). Measurement was performed by using GC-2014 (trade name) manufactured by Shimadzu Corporation with conditions as follows; column: DB-1 (trade name, 30 m x 0.53 mm x 5 µm) manufactured by Agilent Technologies, Inc., temperature of vaporization chamber: 250°C, temperature of detection part: 270°C, temperature program: 50°C (maintained for 5 minutes) → increasing the temperature 10°C/min → 250°C (maintained for 10 minutes), detector: FID, linear speed: 40 cm/s, split ratio: 30, and injection amount: 1 µL.

Further, coloration of the reaction solution was measured by using a UV visible spectrophotometer UV-1700 (trade name) manufactured by Shimadzu Corporation.

### [Example 1]

To a glass flask equipped with a stirrer, a thermometer, and a Dimroth condenser, 2.00 g (13.9 mmol) of 2-naphthol (a compound of the formula [1] in which ring Z is naphthalene, n is 0, and m is 0) which has been purified by crystallization, 2.57g (16.6 mmol) methacrylic anhydride, 5.00 g of toluene, 2.0 mg of 2,6-di-tert-butyl-4-methylphenol (0.10 part by mass relative to 100 parts by mass of 2-naphthol), 2.0 mg of triphenyl phosphite (a compound of the formula [2] in which R² is a phenyl group) (0.10 part by mass relative to 100 parts by mass of 2-naphthol), and 103 mg (1.39 mmol) of lithium carbonate were added followed by reaction under stirring for 3 hours at 80°C. When the reaction is completed, it was confirmed by GC-MS and NMR that 2-naphthyl methacrylate (a compound of the formula [3] in which ring Z is naphthalene, n is 0, m is 0, and R³ is a methyl group) is produced. Yield of 2-naphthyl methacrylate was 98%.

The obtained reaction solution was filtered and the entire reaction solution after the filtration was dissolved in toluene to have 50 mL solution. Then, absorbance was measured by using a quartz cell (1 cm width and height) and a UV visible spectrophotometer. As the absorption spectrum having the maximum value at 540 nm was obtained when high coloration is observed with a naked eye, the comparison was made with the absorbance at 540 nm. The results are shown in Table 1.

### [Examples 2 to 6 and Comparative Examples 1 to 4]

By using the hindered phenol and phosphite that are described in Table 1, the experiments were performed in the same manner as Example 1. The results are shown in Table 1.

**[Table 1]**

| | Hindered phenols | | Other phenols | | Phosphites | | Absorbance (λ = 540 nm) |
|---|---|---|---|---|---|---|---|
| | Compound | Use amount (relative to 2-naphthol) | Compound | Use amount (relative to 2-naphthol) | Compound | Use amount (relative to 2-naphthol) | |
| Example 1 | A1 | 1000 ppm | - | - | B1 | 1000 ppm | 0.015 |
| Example 2 | A1 | 1000 ppm | - | - | B2 | 1000 ppm | 0.035 |
| Example 3 | A1 | 1000 ppm | - | - | B3 | 1000 ppm | 0.029 |
| Example 4 | A1 | 1000 ppm | - | - | B4 | 1000 ppm | 0.030 |
| Example 5 | A2 | 1000 ppm | - | - | B1 | 1000 ppm | 0.025 |
| Example 6 | A1 | 500 ppm | - | - | B1 | 500 ppm | 0.029 |
| Comparative Example 1 | - | - | - | - | - | - | 0.073 |
| Comparative Example 2 | A1 | 1000 ppm | - | - | - | - | 0.072 |
| Comparative Example 3 | - | - | - | - | B1 | 1000 ppm | 0.076 |
| Comparative Example 4 | - | - | X3 | 1000 ppm | B1 | 1000 ppm | 0.083 |

| | | | | | | | |
|---|---|---|---|---|---|---|---|
| Note) A1: 2,6-di-tert-butyl-4-methylphenol A2: 2,6-di-tert-butyl-4-(octadecaneoxycarbonylethyl)phenol 1 X3: 2-tert-butyl-4,6-dimethylphenol B1: triphenyl phosphite B2: tris(2,4-di-tert-butylphenyl)phosphite B3: tridecyl phosphite B4: bis(2,6-di-tert-butyl-4-methylphenyl)pentaerythritol diphosphite | | | | | | | |

[Example 7]

To a glass flask equipped with a stirrer, a thermometer, and a Dimroth condenser, 2.00 g (13.9 mmol) of 2-naphthol (a compound of the formula [1] in which ring Z is naphthalene, n is 0, and m is 0) which has been purified by recrystallization, 2.57g (16.6 mmol) methacrylic anhydride, 5.00 g of toluene, and 55.9 mg (1.39 mmol) of magnesium oxide as a catalyst were added followed by reaction under stirring for 3 hours at 80°C in an air atmosphere. When the reaction is completed, it was confirmed by GC-MS and NMR that 2-naphthyl methacrylate (a compound of the formula [3] in which ring Z is naphthalene, n is 0, m is 0, and R³ is a methyl group) is produced. Yield of 2-naphthyl methacrylate was 100%.

The obtained reaction solution was filtered and the entire reaction solution after the filtration was dissolved in toluene to have 50 mL solution. Then, absorbance was measured by using a quartz cell (1 cm width and height) and a UV visible spectrophotometer. Comparison was made with the absorbance at 417 nm and 540 nm. The results are shown in Table 2.

### [Examples 8 to 13 and Comparative Examples 5 to 7]

The experiments were performed in the same manner as Example 7 except that the type and amount of the catalyst used in Example 7 were modified to the type and amount of the catalyst shown in Table 2. The results are shown in Table 2.

Examples 7 to 9 have low absorbance both at 417 nm and 540 nm. The absorbance at 540 nm was higher in Examples 10 to 13 compared to Examples 7 to 9. However, the absorbance at 417 nm was lower. Meanwhile, Comparative Example 5 has high absorbance both at 417 nm and 540 nm. Although Comparative Examples 6 and 7 have low absorbance at 540 nm, the absorbance at 417 nm was very high, and as a result, the purification load became very high.

**[Table 2]**

| | Catalyst | Catalyst amount (equivalents) | Absorbance (λ = 417 nm) | Absorbance (λ = 540 nm) | Ratio (%) of GC area value after reaction for 1 hour 2-Naphthol/2-naphthyl methacrylate |
|---|---|---|---|---|---|
| Example 7 | Magnesium oxide | 0.1 | 0.058 | 0.005 | 0.41 |
| Example 8 | Magnesium oxide | 0.05 | 0.118 | 0.008 | 1.03 |
| Example 9 | Magnesium oxide | 0.008 | 0.592 | 0.035 | 4.91 |
| Example 10 | Calcium oxide | 0.1 | 0.428 | 0.048 | 2.04 |
| Example 11 | Lithium carbonate | 1.0 | 0.327 | 0.047 | 6.09 |
| Example 12 | Lithium carbonate | 0.5 | 0.478 | 0.086 | 7.85 |
| Example 13 | Sodium carbonate | 0.05 | 0.232 | 0.061 | 3.67 |
| Comparative Example 5 | Lithium carbonate | 0.1 | 1.116 | 0.073 | 15.95 |
| Comparative Example 6 | Magnesium oxide | 0.005 | 1.144 | 0.033 | 30.18 |
| Comparative Example 7 | Magnesium oxide | 0.001 | 1.325 | 0.036 | 62.85 |

### [Example 14]

To a glass flask equipped with a stirrer, a thermometer, and a Dimroth condenser, 2.00 g (13.9 mmol) of 2-naphthol (a compound of the formula [1] in which ring Z is naphthalene, n is 0, and m is 0) which has been purified by crystallization, 2.57g (16.6 mmol) methacrylic anhydride, 5.00 g of toluene, 2.0 mg of 2,6-di-tert-butyl-4-methylphenol (0.10 part by mass relative to 100 parts by mass of 2-naphthol), 2.0 mg of triphenyl phosphite (a compound of the formula [2] in which R² is a phenyl group) (0.10 part by mass relative to 100 parts by mass of 2-naphthol), and 55.9 mg (1.39 mmol) of magnesium oxide were added followed by reaction under stirring for 3 hours at 80°C. When the reaction is completed, it was confirmed by GC-MS and NMR that 2-naphthyl methacrylate (a compound of the formula [3] in which ring Z is naphthalene, n is 0, m is 0, and R³ is a methyl group) is produced. Yield of 2-naphthyl methacrylate was 99%.

The obtained reaction solution was filtered and the entire reaction solution after the filtration was dissolved in toluene to have 50 mL solution. Then, absorbance was measured by using a quartz cell (1 cm width and height) and a UV visible spectrophotometer. As the absorption spectrum having the maximum value at 540 nm was obtained when high coloration is observed with a naked eye, the comparison was made with the absorbance at 540 nm. The results are shown in Table 3.

**[Table 3]**

| | Catalyst | Catalyst amount (equivalents) | Hindered phenol | | Phosphite | | Absorbance (λ =417nm) | Absorbance (λ = 540 nm) | Ratio (%) of GC area value after reaction for 1 hour 2-Naphthol/2-naphthyl methacrylate |
|---|---|---|---|---|---|---|---|---|---|
| | | | Compound | Use amount (relative to 100 parts by mass of 2-naphthyl methacrylate) | Compound | Use amount (relative to 100 parts by mass of 2-naphthyl methaclylate) | | | |
| Example 14 | Magnesium oxide | 0.1 | A1 | 0.10 part by mass | B1 | 0.10 part by mass | 0.016 | 0.002 | 0.57 |

| | | | | | | | | | |
|---|---|---|---|---|---|---|---|---|---|
| Note) A1: 2,6-di-tert-butyl-4-methylphenol B1: triphenyl phosphite | | | | | | | | | |

### [Example 15]

To a glass flask equipped with a stirrer, and a Dimroth condenser, 1.4 g of 2-naphthyl methacrylate (a compound of the formula [3] in which ring Z is naphthalene, n is 0, m is 0, and R³ is a methyl group) containing 2,6-di-tert-butyl-4-methylphenol (0.0024 parts by mass relative to 100 parts by mass of 2-naphthyl methacrylate), triphenyl phosphite (a compound of the formula [2] in which R² is a phenyl group) (0.0024 parts by mass relative to 100 parts by mass of 2-naphthyl methacrylate) was added followed by stirring for 3 hours at 100°C.

After that, the entire amount of 2-naphthyl methacrylate was dissolved in acetonitrile to have a solution of 10 mL and the absorbance was measured by using a quartz cell (1 cm width and height) and a UV visible spectrophotometer. Comparison was made with the absorbance at 417 nm. The results are shown in Table 4.

### [Examples 16 and 17 and Comparative Examples 8 and 9]

The experiments were performed in the same manner as Example 15 except that the type and amount of the anti-oxidant (2,6-di-tert-butyl-4-methylphenol and triphenyl phosphite in Example 15) were modified to the type and amount shown in Table 4. The results are shown in Table 4.

**[Table 4]**

| | 2,6-Di-tert-butyl-4-methylphenol | 4-Methoxyphenol | Triphenyl phosphite | Absorbance (λ = 417 nm) |
|---|---|---|---|---|
| | Use amount (relative to 100 parts by mass of 2-naphthyl methacrylate) | Use amount (relative to 100 parts by mass of 2-naphthyl methacrylate) | Use amount (relative to 100 parts by mass of 2-naphthyl methacrylate) | |
| Example 15 | 0.0024 part by mass | - | 0.0024 part by mass | 0.075 |
| Example 16 | 0.0056 part by mass | - | 0.0056 part by mass | 0.063 |
| Example 17 | 0.020 part by mass | - | 0.020 part by mass | 0.046 |
| Comparative Example 8 | - | - | - | 0.099 |
| Comparative Example 9 | - | 0.0052 part by mass | 0.0051 part by mass | 0.088 |

### [Example 18]

1.00 g of 2-naphthyl methacrylate obtained from Example 14 (a compound of the formula [3] in which ring Z is naphthalene, n is 0, m is 0, and R³ is a methyl group) was dissolved in toluene to have a solution of 10 mL and the absorbance was measured by using a quartz cell (1 cm width and height) and a UV visible spectrophotometer. The results are shown in Fig. 1. Meanwhile, the absorbance at 417 nm was 0.0118. Further, it was found that, in 2-naphthyl methacrylate obtained from Example 14, 2-naphthyl acetate is contained at GC area value ratio of 1.2% (2-naphthyl acetate/2-naphthyl methacrylate x 100).

### [Comparative Example 10]

The experiments were performed in the same manner as Example 18 except that 2-naphthyl methacrylate manufactured by Aldrich Company was used. The results are shown in Fig. 1. Meanwhile, the absorbance at 417 nm was 0.4556. Further, 2-naphthyl acetate was not detected by GC from 2-naphthyl methacrylate manufactured by Aldrich Company, and thus it was found that 2-naphthyl acetate was not contained.

### [Example 19]

By using 2-naphthyl methacrylate obtained from Example 14 (a compound of the formula [3] in which ring Z is naphthalene, n is 0, m is 0, and R³ is a methyl group), the total chlorine content was measured. The total chlorine content was measured as follows; a distillation eluent is combusted by heating it to 100 to 900°C using a sample combustion device QF-02 (trade name, manufactured by Mitsubishi Chemical Corporation), and after allowing the gas to be absorbed in an absorbing pipe, analysis is performed by IC (ion chromatography). The results are shown in Table 5.

### [Comparative Example 11]

The experiments were performed in the same manner as Example 19 except that 2-naphthyl methacrylate manufactured by Aldrich Company is used. The results are shown in Table 5.

**[Table 5]**

| | 2-Naphthyl methacrylate | Total chlorine amount (ppm) |
|---|---|---|
| Example 19 | Obtained from Example 14 | 17 |
| Comparative Example 11 | Manufactured by Aldrich Company | 14000 |

This application claims priority to Japanese Patent Application No. 2013-60459 which has been filed on March 22, 2013 and Japanese Patent Application No. 2013-79070 which has been filed on April 5, 2013, and the entire contents of their disclosure are incorporated herein by reference.

Hereinabove, the present invention is described in view of the embodiments and examples. However, it is evident that the present invention is not limited to those embodiments and examples, and various modifications that can be understood by a person skilled in the art can be made within the constitution or specifically, within the scope of the present invention.

### INDUSTRIAL APPLICABILITY

The present invention relates to a low-tint aryl (meth)acrylate, a production method for the same, and a low-tint aryl (meth)acrylate composition, and an aryl (meth)acrylate with high quality can be produced.

## Claims

1. A production method for an aryl (meth)acrylate represented by the following formula [3] 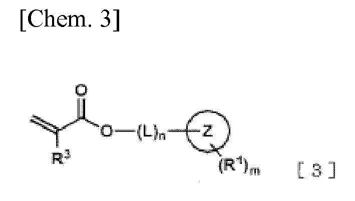 (in the formula, ring Z, L, R¹, n and m have the same meanings as defined in the following formula [1]. R³ represents a hydrogen atom or a methyl group),
by reacting an aromatic compound containing a hydroxy group represented by the following formula [1] with (meth)acrylic acid anhydride 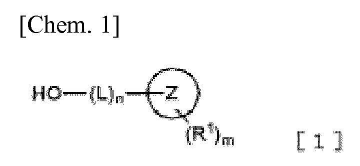 (in the formula, ring Z represents an aromatic hydrocarbon. L represents a divalent linking group. R¹ represents a linear or branched alkyl group, a cycloalkyl group, an aryl group, an aralkyl group, a carboxyl group, an alkoxycarbonyl group, an alkylcarbonyl group, an alkenylcarbonyl group, an arylcarbonyl group, a halogenated alkyl group, an alkoxy group, an aryloxy group, a trialkylsilyl group, a cyano group, a nitro group, a dialkylamino group, a sulfonic acid group, or a halogen atom. n represents an integer of 0 or 1 and m represents an integer of 0 or higher. When m is 2 or higher, R¹ may be the same or different from each other),
in the presence of hindered phenol and phosphite represented by the following formula [2]
[Chem. 2] p-(OR²)₃ [2]
(in the formula, R² represents a linear or branched alkyl group, a cycloalkyl group, an aryl group, an aralkyl group, a carboxyl group, an alkoxycarbonyl group, an alkylcarbonyl group, an alkenylcarbonyl group, an arylcarbonyl group, or a halogenated alkyl group, which may have a substituent group or a hetero bond. R² may be the same or different from each other).

2. The method according to claim 1, wherein the reaction is performed in the presence of a catalyst and the ratio (%) of gas chromatography (GC) area value of the aromatic compound containing a hydroxy group to that of the aryl (meth)acrylate (aromatic compound containing a hydroxy group/aryl (meth)acrylate x 100) is 13% or less after the reaction for 1 hour.

3. The method according to claim 2, wherein the catalyst is at least one of metal oxide and carbonate salt.

4. The method according to claim 2, wherein the catalyst is metal oxide, the reaction temperature is 80°C, and the metal oxide is allowed to be present at 0.006 equivalent or more relative to the aromatic compound containing a hydroxy group such that the ratio (%) of gas chromatography (GC) area value of the aromatic compound containing a hydroxy group to that of the aryl (meth)acrylate (aromatic compound containing a hydroxy group/aryl (meth)acrylate x 100) is 13% or less after the reaction for 1 hour.

5. A production method for an aryl (meth)acrylate represented by the following formula [3] 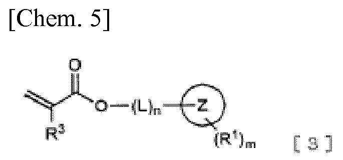 (in the formula, ring Z, L, R¹, n and m have the same meanings as defined in the following formula [1]. R³ represents a hydrogen atom or a methyl group) by reacting an aromatic compound containing a hydroxy group represented by the following formula [1] with (meth)acrylic acid anhydride in the presence of a catalyst, 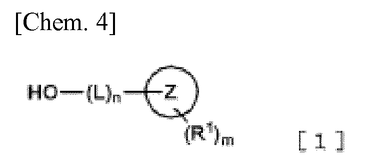 (in the formula, ring Z represents an aromatic hydrocarbon. L represents a divalent linking group. R¹ represents a linear or branched alkyl group, a cycloalkyl group, an aryl group, an aralkyl group, a carboxyl group, an alkoxycarbonyl group, an alkylcarbonyl group, an alkenylcarbonyl group, an arylcarbonyl group, a halogenated alkyl group, an alkoxy group, an aryloxy group, a trialkylsilyl group, a cyano group, a nitro group, a dialkylamino group, a sulfonic acid group, or a halogen atom. n represents an integer of 0 or 1 and m represents an integer of 0 or higher. When m is 2 or higher, R¹ may be the same or different from each other),
wherein the ratio (%) of gas chromatography (GC) area value of the aromatic compound containing a hydroxy group to that of the aryl (meth)acrylate (aromatic compound containing a hydroxy group/aryl (meth)acrylate x 100) is 13% or less after the reaction for 1 hour.

6. The method according to claim 5, wherein the catalyst is at least one of metal oxide and carbonate salt.

7. The method according to claim 5, wherein the catalyst is metal oxide, the reaction temperature is 80°C, and the metal oxide is allowed to be present at 0.006 equivalent or more relative to the aromatic compound containing a hydroxy group such that the ratio (%) of gas chromatography (GC) area value of the aromatic compound containing a hydroxy group to that of the aryl (meth)acrylate (aromatic compound containing a hydroxy group/aryl (meth)acrylate x 100) is 13% or less after the reaction for 1 hour.

8. An aryl (meth)acrylate composition containing hindered phenol and phosphite represented by the following formula [2]
[Chem. 6] P-(OR²)₃ [2]
(in the formula, R² represents a linear or branched alkyl group, a cycloalkyl group, an aryl group, an aralkyl group, a carboxyl group, an alkoxycarbonyl group, an alkylcarbonyl group, an alkenylcarbonyl group, an arylcarbonyl group, or a halogenated alkyl group, which may have a substituent group or a hetero bond. R² may be the same or different from each other),
each at 0.0002 parts by mass or more and 10 parts by mass or less relative to 100 parts by mass of aryl (meth)acrylate represented by the following formula [3] 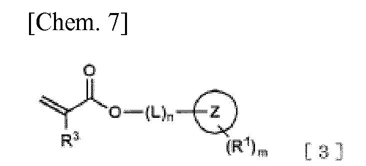 (in the formula, ring Z represents an aromatic hydrocarbon. L represents a divalent linking group. R¹ represents a linear or branched alkyl group, a cycloalkyl group, an aryl group, an aralkyl group, a carboxyl group, an alkoxycarbonyl group, an alkylcarbonyl group, an alkenylcarbonyl group, an arylcarbonyl group, a halogenated alkyl group, an alkoxy group, an aryloxy group, a trialkylsilyl group, a cyano group, a nitro group, a dialkylamino group, a sulfonic acid group, or a halogen atom. n represents an integer of 0 or 1 and m represents an integer of 0 or higher. When m is 2 or higher, R¹ may be the same or different from each other. R³ represents a hydrogen atom or a methyl group).

9. An aryl (meth)acrylate composition containing aryl acetate represented by the following formula [4], 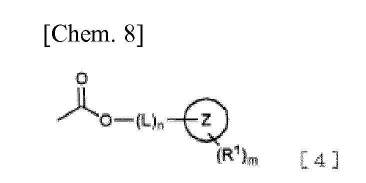 (in the formula, ring Z represents an aromatic hydrocarbon. L represents a divalent linking group. R¹ represents a linear or branched alkyl group, a cycloalkyl group, an aryl group, an aralkyl group, a carboxyl group, an alkoxycarbonyl group, an alkylcarbonyl group, an alkenylcarbonyl group, an arylcarbonyl group, a halogenated alkyl group, an alkoxy group, an aryloxy group, a trialkylsilyl group, a cyano group, a nitro group, a dialkylamino group, a sulfonic acid group, or a halogen atom. n represents an integer of 0 or 1 and m represents an integer of 0 or higher. When m is 2 or higher, R¹ may be the same or different from each other)
and aryl (meth)acrylate represented by the following formula [3] 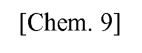 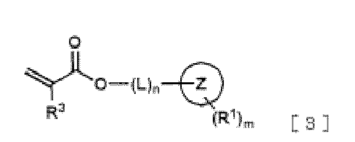 (in the formula, ring Z, L, R¹, n and m have the same meanings as defined in the formula [4]. R³ represents a hydrogen atom or a methyl group),
wherein the ratio (%) of gas chromatography (GC) area value of the aryl acetate to that of the aryl (meth)acrylate (aryl acetate /aryl (meth)acrylate x 100) is 0.01 to 10%.

10. A method for preserving aryl (meth)acrylate by containing, relative to 100 parts by mass of aryl (meth)acrylate represented by the following formula [3], 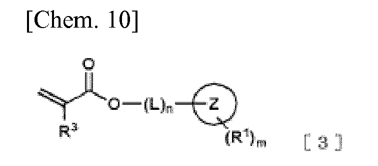 (in the formula, ring Z represents an aromatic hydrocarbon. L represents a divalent linking group. R¹ represents a linear or branched alkyl group, a cycloalkyl group, an aryl group, an aralkyl group, a carboxyl group, an alkoxycarbonyl group, an alkylcarbonyl group, an alkenylcarbonyl group, an arylcarbonyl group, a halogenated alkyl group, an alkoxy group, an aryloxy group, a trialkylsilyl group, a cyano group, a nitro group, a dialkylamino group, a sulfonic acid group, or a halogen atom. n represents an integer of 0 or 1 and m represents an integer of 0 or higher. When m is 2 or higher, R¹ may be the same or different from each other. R³ represents a hydrogen atom or a methyl group),
hindered phenol and phosphite represented by the following formula [2]
[Chem. 11] P-(OR²)₃ [2]
(in the formula, R² represents a linear or branched alkyl group, a cycloalkyl group, an aryl group, an aralkyl group, a carboxyl group, an alkoxycarbonyl group, an alkylcarbonyl group, an alkenylcarbonyl group, an arylcarbonyl group, or a halogenated alkyl group, which may have a substituent group or a hetero bond. R² may be the same or different from each other),
each at 0.0002 parts by mass or more and 10 parts by mass or less.

11. An aryl (meth)acrylate composition containing an aryl (meth)acrylate represented by the following formula [3], wherein a toluene solution of the composition has absorbance of 0.4 or less at a wavelength of 417 nm 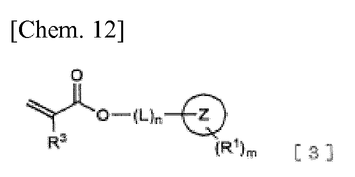 (in the formula, ring Z represents an aromatic hydrocarbon. L represents a divalent linking group. R¹ represents a linear or branched alkyl group, a cycloalkyl group, an aryl group, an aralkyl group, a carboxyl group, an alkoxycarbonyl group, an alkylcarbonyl group, an alkenylcarbonyl group, an arylcarbonyl group, a halogenated alkyl group, an alkoxy group, an aryloxy group, a trialkylsilyl group, a cyano group, a nitro group, a dialkylamino group, a sulfonic acid group, or a halogen atom. n represents an integer of 0 or 1 and m represents an integer of 0 or higher. When m is 2 or higher, R¹ may be the same or different from each other. R³ represents a hydrogen atom or a methyl group).

12. The aryl (meth)acrylate composition according to claim 11, further containing an aryl acetate represented by the following formula [4], 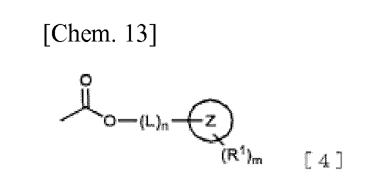 (in the formula, ring Z represents an aromatic hydrocarbon. L represents a divalent linking group. R¹ represents a linear or branched alkyl group, a cycloalkyl group, an aryl group, an aralkyl group, a carboxyl group, an alkoxycarbonyl group, an alkylcarbonyl group, an alkenylcarbonyl group, an arylcarbonyl group, a halogenated alkyl group, an alkoxy group, an aryloxy group, a trialkylsilyl group, a cyano group, a nitro group, a dialkylamino group, a sulfonic acid group, or a halogen atom. n represents an integer of 0 or 1 and m represents an integer of 0 or higher. When m is 2 or higher, R¹ may be the same or different from each other), and
the ratio (%) of gas chromatography (GC) area value of the aryl acetate to that of the a ryl (meth)acrylate (aryl acetate/aryl (meth)acrylate x 100) is 0.01 to 10%.

13. The aryl (meth)acrylate composition according to claim 12, further containing hindered phenol and phosphite represented by the following formula [2]
[Chem. 14] P-(OR²)₃ [2]
(in the formula, R² represents a linear or branched alkyl group, a cycloalkyl group, an aryl group, an aralkyl group, a carboxyl group, an alkoxycarbonyl group, an alkylcarbonyl group, an alkenylcarbonyl group, an arylcarbonyl group, or a halogenated alkyl group, which may have a substituent group or a hetero bond. R² may be the same or different from each other),
each at 0.0002 parts by mass or more and 10 parts by mass or less relative to 100 parts by mass of aryl (meth)acrylate.

14. A n aryl (meth)acrylate composition containing a n aryl (meth)acrylate represented by the following formula [3] and having the total chlorine content of 13000 ppm or less 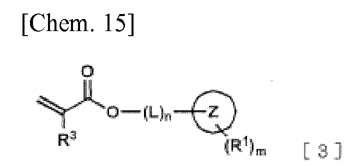 (in the formula, ring Z represents an aromatic hydrocarbon. L represents a divalent linking group. R¹ represents a linear or branched alkyl group, a cycloalkyl group, an aryl group, an aralkyl group, a carboxyl group, an alkoxycarbonyl group, an alkylcarbonyl group, an alkenylcarbonyl group, an arylcarbonyl group, a halogenated alkyl group, an alkoxy group, an aryloxy group, a trialkylsilyl group, a cyano group, a nitro group, a dialkylamino group, a sulfonic acid group, or a halogen atom. n represents an integer of 0 or 1 and m represents an integer of 0 or higher. When m is 2 or higher, R¹ may be the same or different from each other. R³ represents a hydrogen atom or a methyl group).

15. The aryl (meth)acrylate composition according to claim 14, further containing an aryl acetate represented by the following formula [4], 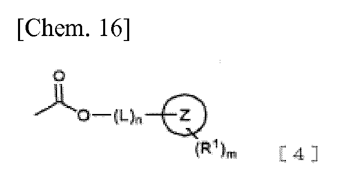 (in the formula, ring Z represents an aromatic hydrocarbon. L represents a divalent linking group. R¹ represents a linear or branched alkyl group, a cycloalkyl group, an aryl group, an aralkyl group, a carboxyl group, an alkoxycarbonyl group, an alkylcarbonyl group, an alkenylcarbonyl group, an arylcarbonyl group, a halogenated alkyl group, an alkoxy group, an aryloxy group, a trialkylsilyl group, a cyano group, a nitro group, a dialkylamino group, a sulfonic acid group, or a halogen atom. n represents an integer of 0 or 1 and m represents an integer of 0 or higher. When m is 2 or higher, R¹ may be the same or different from each other), and
the ratio (%) of gas chromatography (GC) area value of the aryl acetate to that of the a ryl (meth)acrylate (aryl acetate/aryl (meth)acrylate x 100) is 0.01 to 10%.
